# EUROPÄISCHE PATENTSCHRIFT

(11) **EP 2 582 290 B1**
(45) Veröffentlichungstag und Bekanntmachung des Hinweises auf die Patenterteilung: **14.05.2014**
(21) Anmeldenummer: 11728608.8
(22) Anmeldetag: 17.06.2011
(51) Int. Cl.: A61B 5/04, A61B 5/0408, A61B 5/0424, A61B 5/0478, A61B 5/0492, A61N 1/04, A41D 13/12, A61N 1/30

(54) **BEFEUCHTETE SENSORKONTAKTEINHEIT**
MOISTENED SENSOR CONTACT UNIT
BLOC DE CONTACT A CAPTEUR HUMIDIFIE

(30) Priorität: 17.06.2010 EP 10166405
(43) Veröffentlichungstag der Anmeldung: 24.04.2013
(73) Patentinhaber: EMPA Eidgenössische Materialprüfungs- und Forschungsanstalt, 8600 Dübendorf (CH)
(72) Erfinder: WEDER, Markus, CH-9230 Flawil (CH)
(74) Vertreter: Schmauder & Partner AG Patent- & Markenanwälte VSP
(86) Internationale Anmeldenummer: PCT/EP2011/060107
(87) Internationale Veröffentlichungsnummer: WO 2011/157821

(56) Entgegenhaltungen:
- EP-A1- 0 510 786
- EP-A1- 2 322 710
- WO-A2-2004/017829
- US-B1- 6 263 226

## Beschreibung

### Technisches Gebiet

Die Erfindung betrifft eine Sensorkontakteinheit zur Messung von elektrischen Signalen eines Körpers, insbesondere eines menschlichen Körpers. Weiterhin betrifft die Erfindung einen Brustgurt mit einer entsprechenden Sensorkontakteinheit, insbesondere zur Messung von EKG-Daten.

### Stand der Technik

Die Messung von elektrischen Körpersignalen wie z.B. EKG (Elektrokardiogramm), EEG (Elektro-Enzephalogramm), EMG (Elektromyogramm) sind weit verbreitete Messmethoden zur Ableitung von Körpersignalen. Bisher wird das EKG im medizinischen Bereich praktisch ausschliessliich mit den Ag/AgCl Gelelektroden gemessen, welche um den Brustbereich auf die Haut geklebt wurden. Diese Elektroden bilden quasi einen Standard und werden weltweit angewendet, wenn es um EKG Messungen im medizinischen Bereich geht. Der Nachteil ist dass diese Elektroden nur eine limitierte Zeit von bis zu ca. 24 h funktionieren. Danach trocknen sie aus und ergeben kein geeignetes Signal mehr. Es hat sich jedoch herausgestellt, dass auch Langzeitmessungen wesentlich sein können. Dabei ist es wichtig, auch über mehere Tage das EKG mittels spezieller Holter - tragbare Logger - erfassen zu können. Dazu sind neue trockene Elektroden auf dem Markt aufgetaucht, die für EKG Messungen geeignet sein sollen. Beispielsweise sind Stickelektroden und Sensor-Shirts bekannt. Solche Elektroden funktionieren bei einer körperlichen Aktivität gut und ergeben gute Signale, insbesondere wenn die Person schwitzt und somit eine feuchte Haut hat, aber eben nur solange die Haut feucht ist. Sobald aber die Elektroden trocken sind ist keine Messung mehr möglich. Der gesamte medizinische Bereich wo keine feuchte, sondern eine trockene Haut vorliegt, kann mit diesen Elektroden langzeitig nicht gemessen werden. Gerade dieser Bereich wäre aber aus wissenschaftlicher und medizinischer Sicht sehr interessant, z.B. die Ueberwachung von Herzrisikopatienten oder generell bei älteren Leuten wo normalerweise eine trockene Haut vorliegt.

Ein weiteres Problem bei trockenen Elektroden sind die Bewegungsartefakte und die relativ hohe Pressung der Elektrode auf die Haut. Bei trockener Haut ist das Bewegen noch viel kritischer, d.h. die geringste Bewegung der Arme oder Beine kann zu extrem hohen Artefakten führen und aus diesen ein vernünftiges EKG-Signal herauszufiltern ist extrem schwierig.

Im Zusammenhang mit der Überwachung des Menschen können mit solchen Systemen wo eine Langzeiterfassung des EKG möglich ist auch Schlafversuche durchgeführt werden wo über die HRV Chaostheorie die Tiefschlafphasen exakt erkannt werden können oder z.B. als Sensor für die Stresserkennung von Feuerwehrleuten oder Piloten, Lastwagenfahrer etc..

Als Beispiele für den Stand der Technik seien hier die EP-A-0 011 813 aufgeführt, bei der ein flexibles Kissen mit Chemikalie zur Abgabe an die Haut vorgeschlagen wird, wobei die Unterseite zur Haut eine permeable Membrane aufweisen soll. Nachteilig daran ist, dass die Befeuchtung von der Hautseite kommt und dass der Vorschlag eine Chemikalie zwingend erfordert. Weiterhin erscheint die EP 0 409 067 A2 als Hintergrundinformation interessant, bei der eine benetzbare Elektrode mit Flüssigkeitsspeicherndem Absorbermaterial vorgeschlagen wird, wobei ein Absorber mit ionischer Flüssigkeit zwischen der Elektrode und der Haut vorgesehen ist. In der US 5 057 072 A wird eine Elektrode mit Reservoir mit einer Chemikalie bzw. ionischen Flüssigkeit vorgeschlagen, wobei die Flüssigkeit auf der Hautseite ist und sich dahinter die Elektrode befindet. Auch hier erscheint es nachteilig, dass die Befeuchtung von der Hautseite kommt.

Aus der US 6263226 B1 ist eine Wegwerfelektrode bekannt, welche nach 8 bis 12 h entsorgt wird. Dieses ist unzweckmässig. Für die Elektroden gemäss der Aufgabe der Erfindung ist ein wochenlanger Dauereinsatz gefordert. Weiterhin ist zwischen der Haut und der eigentlichen elektrischen Elektrode in der US 6263226 B1 ein elektrisch leitendes Gel erforderlich. Gemäss der Aufgabe der Erfindung soll die (Stick)-Elektrode direkt auf der Haut aufliegen und es kein Gel notwendig resp. kein Schwamm zwischen der Haut der Elektrode erforderlich sein.

Aus der WO 2004/017829 A1 sind Elektroden bekannt, die für Impedanz-Tomography vorgesehen sind. Für Elektrokardiographie gemäss der Aufgabe der Erfindung erscheinen diese Elektroden ungeeignet. Wiederum ist zwischen der Elektrode und der Haut ein Gel vorgesehen, welches es - gemäss der Aufgabe der Erfindung - wegen des Dauereinsatzes zu vermeiden gilt. Bei der Elektrode der WO 2004/017829 A1 ist die Befeuchtung von hinten vorgesehen, jedoch unvorteilhafterweise mit Ionen. Wie vorstehend beschrieben, soll - gemäss der Aufgabe der Erfindung - der Verdunstungsgenerator nachgefüllt werden können, während derjenige in der WO 2004/017829 A1 komplett versiegelt und nach Gebrauch weggeworfen werden muss.

Aus der EP 510 786 A1 ist eine Elektrode bekannt, die auf einem Hydrogellayer beruht, welcher in Kombination mit einer nicht leitenden Materialschicht zusammengebracht ist und an eine wiederverwendbare Klammer angeschlossen wird. Es ist keine Befeuchtungseinrichtung vorgesehen. Die Vorrichtung der EP 510 786 A1 beruht auf dem Prinzip eines Elektrolytlayers auf der Basis von Hydrogel. Aus den vorstehend genannten Gründen soll dies erfindungsgemäss vermieden werden.

### Darstellung der Erfindung

Aufgabe der Erfindung ist es demnach, eine verbesserte Sensorkontakteinheit vorzuschlagen, bei der die beschriebenen Nachteile vom Stand der Technik zumindest vermieden werden können.

Diese Aufgabe wird gelöst durch die Sensorkontakteinheit zum Herstellen und Aufrechterhalten eines elektrischen Kontaktes zwischen der Haut einer Person und einer elektrischen Einheit gemäss Anspruch 1. Die Sensorkontakteinheit ist besonders geeignet zur Messung von elektrischen Daten der Person und umfasst eine erste textile Schicht, in oder auf der elektrisch leitende Fäden, vorzugsweise aus Metall oder leitfähigen Kunststoffgarnen, angeordnet sind, ein flächiges Befeuchtungselement, welches auf der textilen Schicht aufliegt, einen elektrischen Anschluss, der mit der textilen Schicht in elektrischem Kontakt steht, und ein Flüssigkeitsreservoir. Das flächige Befeuchtungselement umfasst zumindest eine Innenschicht, eine Aussenschicht sowie einen dazwischen angeordneten Befeuchtungsbereich. Die Innenschicht ist aus einem wasserdampfdurchlässigen Material gebildet und der Befeuchtungsbereich ist mit dem Flüssigkeitsreservoir zur Versorgung des Befeuchtungsbereichs in Verbindung bringbar. Die vorliegende Erfindung beruht auf einer definierten, dampfförmigen Abgabe von Feuchtigkeit an die Elektroden. Damit ist die Erfindung - im Gegensatz zu den Einrichtungen gemäss dem Stand der Technik - durch die Materialauswahl und den Aufbau geeignet, die Aggregatzustände vorteilhaft auszunützen, nämlich die langsame und definierte Abgabe von Wasserdampf. Diese langsame und definierte Abgabe von Wasserdampf geschieht von hinten, also von der den Elektroden abgewandten Seite und wird mittels eines speziellen Befeuchtungselementes ermöglicht, welches mit einem flexiblen Tank gespeist wird. Dabei haben die Massnahmen der Erfindung zunächst einmal zur Folge, dass langzeitig und unabhängig von chemischen Zusätzen, die als Hilfsmittel allerdings nicht grundsätzlich ausgeschlossen sind, die Sensorkontakteinheit guten Kontakt für die elektrischen Personendaten bereitstellt. Die Innenschicht ist zwar wasserdampfdurchlässig, aber erfindungsgemäss wasserdicht ausgebildet, um ein Auslaufen der Flüssigkeit zu verhindern.

Wasserdampfdurchlässigkeit ist mit einem Wasserdampfdurchgangswiderstand Rₑₜ gemäss ISO 11092 definiert und beträgt im Sinne dieser Erfindung einen Wert < 20 m²Pa/W, während die Wasserdichtigkeit nach EN 20811 definiert ist und einen Wert von > 1300 mm Wassersäule betragen soll.

Textile Materialien, die solche Werte in Bezug auf die Wasserdampfdurchlässigkeit und die Wasserdichtigkeit aufweisen, sind bekannt, wobei hier - nur beispielhaft - auf als Membranen ausgebildete Textilien aus Polyetherester, einer Verkettung von Polyester- und Polyethermolekülen hingewiesen wird, wobei für die vorliegende Anwendung die so genannten atmungsaktiven Eigenschaften mit einem Wasserdampfdurchgangswiderstand gemäss ISO 11092 von Rₑₜ < 6 m²Pa/W nicht erforderlich erscheint. Ein weiteres Beispiel solcher wasserdampfdurchlässiger aber wasserdichter Materialien ist als textile Polyfluourethenmembran bekannt (im Handel z.B. unter dem Namen Goretex® erhältlich).

Es sollte an dieser Stelle nochmals auf den Unterschied zwischen der Eigenschaft der Schicht bezüglich der Permeation bezüglich Wasserdampf und der Dichtheit dieser Membrane gegenüber flüssigem Wasser hingewiesen werden.

Bevorzugte Ausgestaltungen der Erfindung sind in den abhängigen Ansprüchen definiert.

Die Innenschicht kann auch hydrophob und der Befeuchtungsbereich hydrophil ausgebildet sein, so dass ein Saugeffekt durch den hydrophilen Befeuchtungsbereich des Befeuchtungselements erzielt wird. Hydrophob heisst, dass die Aussenseite der zum Körper geneigten Schicht hydrophob sein kann, z.B. durch eine geeignete Membrane und die Schicht selber wiederum wasserdampfdurchlässig ist. Dies ist kein Widerspruch und ist bei den zuvor beschriebenen mikroporösen Membranen anzutreffen. Ebenfalls kann z.B. die Aussenseite einer hydrophilen, wasserdampfdurchlässigen Membrane mittels Plasmabehandlung hydrophobisiert werden. Die Aussenschicht kann vorteilhafterweise wasserdicht ausgebildet und zudem auch wasserdampfundurchlässig sein, jeweils mit den zuvor verwendeten Werten.

Für die Innenschicht wird eine Dicke von 1 bis 50 µm angegeben, wobei vorzugsweise Dicken von 5 bis 15 µm zum Einsatz kommen werden.

Das Befeuchtungselement ist vorteilhafterweise an den Seiten wasserdicht ausgebildet, vorzugsweise durch Verschweissen der Innenschicht mit der Aussenschicht im Bereich ihres Aussenbereichs.

Besonders vorteilhaft ist die Sensorkontakteinheit, wenn der Befeuchtungsbereich des Befeuchtungselementes flächige, kanalartige Strukturen aufweist, die von einer Flüssigkeit aus dem Flüssigkeitsreservoirs durchströmbar sind. Zur Versorgung ist eine in der Innen- und oder der Aussenschicht angebrachte, flächige Entlüftungsmembrane vorteilhaft, welche mit den Kanälen in Verbindung steht. Alternativ dazu ist ein in der Innen- und oder der Aussenschicht angebrachtes, flächiges Entlüftungsventil. Die flächige Ausgestaltung der eingeprägten kanalartigen Struktur ist vorzugsweise als Spiralen oder mäanderförmige Strukturen ausgebildet. Die Dicken der kanalartigen Struktur können variieren, wobei vorzugsweise die zuführenden, dem Flüssigkeitsreservoir näherstehenden Kanäle etwas dicker als die abführenden, der Entlüftungsmembran näherstehenden Kanäle sind.

Das Flüssigkeitsreservoir umfasst bevorzugt einen flexiblen Tank, vorzugsweise aus z.B. Polyäthylen oder PVC.

Die Sensorkantakteinheit umfasst in einer bevorzugten Ausgestaltung ein textiles Trägermaterial, welches sich flächig an die Aussenschicht anschliesst.

Gemäss einem weiteren Aspekt der vorliegenden Erfindung ist ein Brustsensor mit einer Sensorkontakteinheit gemäss dem ersten Aspekt ausgestattet und umfasst einen Brustgurt. Das Flüssigkeitsreservoir ist dann mittels eines flexiblen Tanks ausgebildet und in den Gurt integriert, sodass durch die leichte Pressung des Gurts auf den Tank ein Druck ausgeübt werden kann, der die Befeuchtung des Befeuchtungsbereichs bewirkt oder unterstützt.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Ausführungsbeispiele der Erfindung werden nachfolgend anhand der Zeichnungen näher beschrieben, dabei zeigen:
- Fig. 1: einen Schichtaufbau einer Sensorkontakteinheit gemäss einem bevorzugten Ausführungsbeispiel der vorliegenden Erfindung;
- Fig. 2: ein Befeuchtungselement nach Figur 1 in Draufsicht, schematisch.

### Wege zur Ausführung der Erfindung

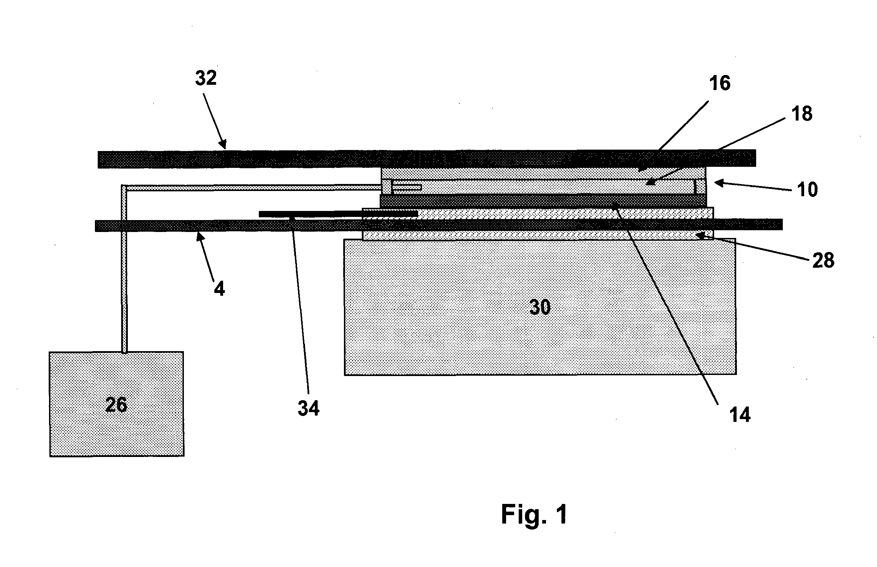

Die in Figur 1 in ihrer Schichtung dargestellte Sensorkontakteinheit 2 umfasst eine erste textile Schicht 4, in oder auf der elektrisch leitende Elemente, insbesondere Leiterfäden - in dem in Figur 1 gezeigten Ausführungsbeispiel als Stickelektroden 28 - angeordnet sind, ein flächiges Befeuchtungselement 10, welches auf der textilen Schicht 4 aufliegt, einen elektrischer Anschluss 34, der mit der ersten textilen Schicht 4 in elektrischem Kontakt steht, und ein Flüssigkeitsreservoir 26. Das flächige Befeuchtungselement 10 umfasst zumindest eine Innenschicht 14, eine Aussenschicht 16 sowie einen dazwischen angeordneten Befeuchtungsbereich 18. Die Innenschicht 14 ist im vorliegenden Ausführungsbeispiel aus einem wasserdampfdurchlässigen, aber wasserundurchlässigem Material gebildet und der Befeuchtungsbereich 18 steht mit dem Flüssigkeitsreservoir 26 zur Versorgung des Befeuchtungsbereichs mittels eines Zuführschlauches 24 in Verbindung. Die Innenschicht ist zwar wasserdampfdurchlässig ausgebildet, ist aber wasserdicht, um ein Auslaufen der Flüssigkeit zu verhindern. Die Aussenschicht ist im vorliegenden Ausführungsbeispiel wasserdicht und zudem auch wasserdampfundurchlässig. Im vorliegenden Ausführungsbeispiel wird als Innenschicht 14 sowie als Aussenschicht 16 als Membranen ausgebildete Textilien aus Polyetherester verwendet, die im Handel (z.B. unter dem Namen Sympatex®) erhältlich ist. Die Innenschicht 14 hat im vorliegenden Ausführungsbeispiel eine Dicke von ca. 5 bis 15 µm. Sie ist wasserdampfdurchlässig mit einem Wasserdampfdurchgangswiderstand Rₑₜ gemäss ISO 11092 < 20 m²PaA/W, während die Wasserdichtigkeit nach EN 20811 der Innenschicht einen Wert von > 1300 mm Wassersäule hat.

Gewisse Polymere/Membranen sind von Hause aus hydrophob oder hydrophil, Polyether-esther (Sympatex®) sind hydrophil, Polyfluourethenmembranen(Gore-Tex®) hingegen ist hydrophob. Zum besseren Verständnis wird hier noch angegeben, dass es folgende Möglichkeiten gibt, ein Gewebe hydrophob oder hydrophil auszurüsten, nämlich eine Plasmabeschichtung, bei der für die Eigenschaft "hydrophob" eine Gasmischung aus Fluorkohlenstroff Monomer oder Silikonmonomer verwendet wird, bzw. "hydrophil", bei der eine Gasmischung aus Kohlenwasserstoffmonomer plus Sauerstoff oder stickstoffhaltigem Monomer verwendet wird, sowie eine nasschemische Ausrüstung, nämlich Hydrophobierungen mit Fluorkarbonharzen oder Silikonen bzw. eine Hydrophilisierung mit Netzmittel aus Tensidebasis.

Das Befeuchtungselement 10 ist im Ausführungsbeispiel an den Seiten wasserdicht ausgebildet, vorzugsweise durch Verschweissen der Innenschicht mit der Aussenschicht 16 im Bereich ihres Aussenbereichs.

Im vorliegenden Ausführungsbeispiel weist der Befeuchtungsbereich 18 des Befeuchtungselementes 10 flächige, kanalartige Strukturen auf, die von der Flüssigkeit aus dem Flüssigkeitsreservoir 26 durchströmbar sind. Im Ausführungsbeispiel ist in der Aussenschicht eine flächige Entlüftungsmembrane 20 angebracht, welche mit den Kanälen in Verbindung steht. Alternativ dazu ist ein in der Innen- und oder der Aussenschicht angebrachtes, flächiges Entlüftungsventil. Die flächige Ausgestaltung der eingeprägten kanalartigen Struktur ist im vorliegenden Ausführungsbeispiel als Spiralen -alternativ als mäanderförmige Strukturen - ausgebildet. Die Dicken der kanalartigen Struktur variieren, wobei die zuführenden, dem Flüssigkeitsreservoir 26 näherstehenden Kanäle etwas dicker als die abführenden, der Entlüftungsmembran 20 näherstehenden Kanäle sind.

Das Flüssigkeitsreservoir 26 umfasst bevorzugt einen flexiblen Tank, im Ausführungsbeispiel aus Polyäthylen.

Die Sensorkontakteinheit 2 umfasst im Ausführungsbeispiel ein textiles Trägermaterial 32, welches sich flächig an die Aussenschicht 16 anschliesst. Im vorliegenden Ausführungsbeispiel bildet dieses textile Trägermaterial 32 einen Brustgurt aus. Das Flüssigkeitsreservoir 26 ist dabei mittels eines flexiblen Tanks ausgebildet und in den Gurt integriert, sodass durch die leichte Pressung des Gurts auf den Tank ein Druck ausgeübt werden kann, der die Befeuchtung des Befeuchtungsbereichs bewirkt oder zumindest unterstützt.

Die Leitfähigkeit ist - wie in Figur 1 dargestellt - dadurch bewirkt, dass in die erste textile Schicht 4 Leiterfäden zur Ausbildung einer Stickelektrode 28 eingestickt sind, die durch die erste textile Schicht 4 hindurch gehen.

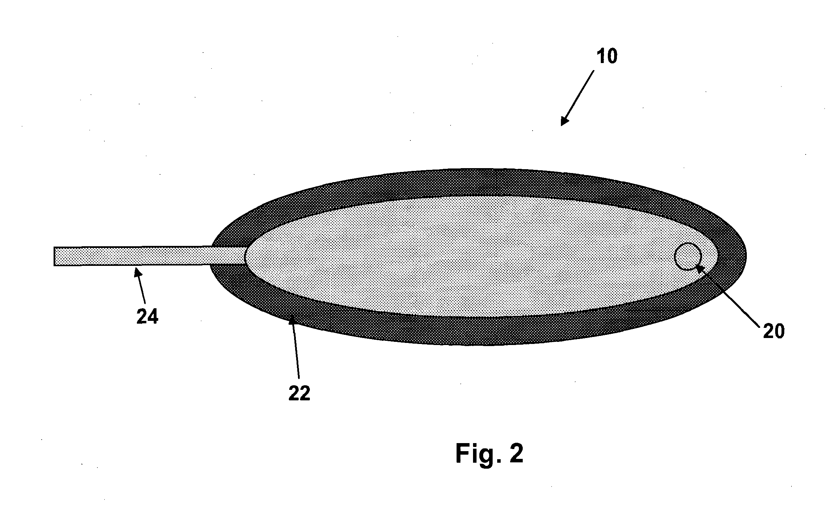

Durch die in Figur 2 dargestellte, praktisch luftdichte Randabschliessung des Befeuchtungselements 10 entsteht beim Verdunsten von Wasser ein Unterdruck, welcher bewirkt, dass Wasser vom Reservoir in das Befeuchtungselement gelangt. Es entsteht als ein Saugeffekt analog dem Blattspalt bei Baumblättern.

Die Befüllung des Tanks mit destilliertem Wasser erfolgt im Ausführungsbeispiel mittels einer medizinischen Spritze und einem speziellen Verschluss mit Rückschlagventil in der Zuleitung von Tank zu Befeuchtungselementen.

Zur Verhinderung von Algen, Bakterien und Pilzen wird dem destilliertem Wasser noch ein Zusatz eines Bakterizids zugefügt.

### Bezugszeichenliste

- 2: Sensorkontakteinheit
- 4: erste textile Schicht
- 10: Befeuchtungselement
- 14: Innenschicht
- 16: Aussenschicht
- 18: Befeuchtungsbereich
- 20: Entlüftungsmembrane
- 22: Randverschweissung
- 24: Zuführschlauch
- 26: Flüssigkeitsreservoir
- 28: Stickelektrode
- 30: Haut
- 32: textiles Trägermaterial
- 34: elektrischer Anschluss

## Patentansprüche

1. Sensorkontakteinheit (2) zum Herstellen und Aufrechterhalten eines elektrischen Kontaktes zwischen der Haut (30) einer Person und einer elektrischen Einheit, insbesondere eines elektrischen Messsensors zur Messung von elektrischen Daten der Person, wobei die Sensorkontakteinheit umfasst
- eine erste textile Schicht (4), in oder auf der elektrisch leitende Fäden (28), vorzugsweise aus Metall oder metallisierten Kunststoffgarnen, angeordnet sind,
- ein flächiges Befeuchtungselement (10), welches auf der textilen Schicht (4) aufliegt,
- einen elektrischer Anschluss (34), der mit der ersten textilen Schicht (4) in elektrischem Kontrakt steht, und
- ein Flüssigkeitsreservoir (26),
wobei das flächige Befeuchtungselement (10) zumindest eine auf der ersten textilen Schicht (4) aufliegende Innenschicht (14), eine Aussenschicht (16) sowie einen dazwischen angeordneten Befeuchtungsbereich (18) aufweist und der Befeuchtungsbereich (18) mit dem Flüssigkeitsreservoir (26) zur Versorgung des Befeuchtungsbereichs (18) in Verbindung bringbar ist, **dadurch gekennzeichnet, dass** die Innenschicht (14) aus einem wasserdampfdurchlässigen und bezüglich flüssigem Wasser wasserdichten Material gebildet ist.

2. Sensorkontakteinheit nach Anspruch 1, **dadurch gekennzeichnet, dass** die wasserdampfdurchlässige, wasserdichte Innenschicht (14) einen Wasserdampfdurchgangswiderstand Rₑₜ von < 20 m²Pa/W und eine Wasserdichtigkeit von > 1300 mm aufweist.

3. Sensorkontakteinheit nach Anspruch 1 oder 2, **dadurch gekennzeichnet, dass** die der ersten textilen Schicht (4) zugewandte Seite der Innenschicht (14) hydrophob und der Befeuchtungsbereich (18) hydrophil ausgebildet ist, so dass ein Saugeffekt durch den hydrophilen Befeuchtungsbereich (18) des Befeuchtungselements (10) erzielt wird.

4. Sensorkontakteinheit nach einem der Ansprüche 1 bis 3, **dadurch gekennzeichnet, dass** die Aussenschicht (16) wasserdicht mit einem Wert von > 1300 mm, vorzugsweise auch wasserdampfundurchlässig mit einem Wasserdampfdurchgangswiderstand Rₑₜ von > 20 m²Pa/W, ausgebildet ist.

5. Sensorkontakteinheit nach einem der Ansprüche 1 bis 4, **dadurch gekennzeichnet, dass** die Innenschicht (14) eine Dicke von 1 bis 50 µm, vorzugsweise 5 bis 15 µm aufweist.

6. Sensorkontakteinheit nach einem der Ansprüche 1 bis 5, **dadurch gekennzeichnet, dass** das Befeuchtungselement (10) an dessen Seiten wasserdicht ausgebildet ist, vorzugsweise durch Verschweissen der Innenschicht (14) mit der Aussenschicht (16) im Bereich ihres Aussenbereichs (22).

7. Sensorkontakteinheit nach einem der Ansprüche 1 bis 6, **dadurch gekennzeichnet, dass** der Befeuchtungsbereich (18) des Befeuchtungselementes (10) flächige, kanalartige Strukturen aufweist, die von einer Flüssigkeit aus dem Flüssigkeitsreservoir (26) durchströmbar sind.

8. Sensorkontakteinheit nach Anspruch 7, **gekennzeichnet durch** eine in der Innen- und oder der Aussenschicht (14, 16) angebrachte, flächige Entlüftungsmembrane (20) oder ein flächiges Entlüftungsventil, welche jeweils mit den Kanälen in Verbindung steht.

9. Sensorkontakteinheit nach einem der Ansprüche 7 oder 8, **dadurch gekennzeichnet, dass** die flächige Ausgestaltung der eingeprägten kanalartigen Struktur als Spiralen oder mäanderförmige Strukturen ausgebildet ist.

10. Sensorkontakteinheit nach einem der Ansprüche 7 bis 9, **dadurch gekennzeichnet, dass** die Dicken der kanalartigen Struktur variieren, wobei vorzugsweise die zuführenden, dem Flüssigkeitsreservoir näherstehenden Kanäle etwas dicker als die abführenden, der Entlüftungsmembran (20) bzw. des Entlüftungsventils näherstehenden Kanäle sind.

11. Sensorkontakteinheit nach einem der Ansprüche 1 bis 10, **dadurch gekennzeichnet, dass** das Flüssigkeitsreservoir (26) einen flexiblen Tank, vorzugsweise aus z.B. Polyäthylen oder PVC aufweist.

12. Sensorkontakteinheit nach einem der Ansprüche 1 bis 11, **gekennzeichnet durch** ein textiles Trägermaterial (32), welches sich flächig an die Aussenschicht anschliesst.

13. Brustsensor mit einer Sensorkontakteinheit nach einem der Ansprüche 1 bis 12 und einem Brustgurt, wobei das Flüssigkeitsreservoir (26) mittels eines flexiblen Tanks ausgebildet ist und in den Gurt integriert ist, sodass durch die leichte Pressung des Gurts auf den Tank ein Druck ausgeübt werden kann, der die Befeuchtung des Befeuchtungsbereich (8) bewirkt oder unterstützt.

## Claims

1. Sensor contact unit (2) for establishing and maintaining an electrical contact between the skin (30) of a person and an electrical unit, particularly an electrical measuring sensor for measuring electrical data of the person, wherein the sensor contact unit comprises
- a first textile layer (4), in which or on which there are arranged electrically conductive threads (28), preferably made of metal or metallized synthetic yarns,
- a planar humidification element (10) lying upon the textile layer (4),
- an electrical connector (34) being in electrical contact with the first textile layer (4), and
- a fluid reservoir (26),
wherein the planar humidification element (10) comprises at least an inner layer (14) lying upon the first textile layer (4), an outer layer (16), and a humidification domain (18) arranged therebetween, and wherein the humidification domain (18) can be brought into communication with the fluid reservoir (26) for supplying the humidification domain (18), **characterized in that** the inner layer (14) is made of a material that is water vapor permeable and water impermeable with respect to liquid water.

2. Sensor contact unit according to claim 1, **characterized in that** the water vapor permeable, water impermeable inner layer (14) has a water vapor transfer resistance Rₑₜ of < 20 m²Pa/W and a water impermeability of > 1300 mm.

3. Sensor contact unit according to claim 1 or 2, **characterized in that** the side of the inner layer (14) facing the first textile layer (4) is configured hydrophobically and that the humidification domain (18) is configured hydrophilically, so that a suction effect is achieved by the hydrophilic humidification domain (18) of the humidification element (10).

4. Sensor contact unit according to one of claims 1 to 3, **characterized in that** the outer layer (16) is configured water impermeably with a value of > 1300 mm, preferably also water vapor impermeably with a water vapor transfer resistance Rₑₜ of > 20 m²Pa/W.

5. Sensor contact unit according to one of claims 1 to 4, **characterized in that** the inner layer (14) has a thickness of 1 to 50 µm, preferably of 5 to 15 µm.

6. Sensor contact unit according to one of claims 1 to 5, **characterized in that** the humidification element (10) is configured water impermeably at the sides thereof, preferably by welding of the inner layer (14) with the outer layer (16) in the region of their outer domain (22).

7. Sensor contact unit according to one of claims 1 to 6, **characterized in that** the humidification domain (18) of the humidification element (10) comprises planar, channel-like structures which are configured to allow flowthrough by a liquid from the fluid reservoir (26).

8. Sensor contact unit according to claim 7, **characterized by** a planar venting membrane (20) being arranged in the inner layer and/or in the outer layer (14, 16) or by a planar venting valve, each being in contact with the channels.

9. Sensor contact unit according to one of claims 7 or 8, **characterized in that** the planar embodiment of the stamped-in channel-like structure is configured as spirals or as meander-like structures.

10. Sensor contact unit according to one of claims 7 to 9, **characterized in that** the thicknesses of the channel-like structures have variations, preferably with the supplying channels positioned closer to the fluid reservoir are slightly thicker than the channels conducting away that are closer to the the venting membrane (20) or to the venting valve, as the case may be.

11. Sensor contact unit according to one of claims 1 to 10, **characterized in that** the fluid reservoir (26) comprises a flexible tank, preferably made of, for example, polyethylene or PVC.

12. Sensor contact unit according to one of claims 1 to 11, **characterized by** a textile support material (32) that is laminarly adjacent to the outer layer.

13. Chest sensor with a sensor contact unit according to one of claims 1 to 12 and with a chest strap, wherein the fluid reservoir (26) is configured by means of a flexible tank and is integrated into the strap so that by means of a slight compression of the strap a pressure may be applied to the tank which causes or supports the humidification of the humidification domain (8).

## Revendications

1. Unité de contact de capteur (2) pour créer et maintenir un contact électrique entre la peau (30) d'une personne et une unité électrique, en particulier un capteur de mesure électrique pour permettre la mesure de données électriques de la personne, cette unité de contact de capteur comprenant :
- une première couche textile (4) située dans ou sur des fibres électriquement conductrices (28) de préférence en métal ou en des fils synthétiques métallisés,
- un élément d'humidification plan (10) qui est appliqué sur la couche textile (4),
- une connexion électrique (34) qui est en contact électrique avec la première couche textile (4), et
- un réservoir de liquide (26),
dans laquelle l'élément d'humidification plan (10) comporte au moins une couche interne (14) appliquée sur la première couche textile (4), une couche externe (16) ainsi qu'une zone d'humidification (18) positionnée entre ces couches, et la zone d'humidification (18) peut être mise en liaison avec le réservoir de liquide (26) pour permettre l'alimentation de la zone d'humidification (18),
**caractérisée en ce que**
la couche interne (14) est réalisée en un matériau perméable à la vapeur d'eau et étanche vis-à-vis de l'eau liquide.

2. Unité de contact conforme à la revendication 1,
**caractérisée en ce que**
la couche interne (14) perméable à la vapeur d'eau et étanche à l'eau présente une résistance au passage de la vapeur d'eau Rₑₜ < 20 m²Pa/W et une étanchéité à l'eau > 1300 mm.

3. Unité de contact conforme à la revendication 1 ou 2,
**caractérisée en ce que**
la face de la couche interne (14) tournée vers la première couche textile (4) est hydrophobe et la zone d'humidification (18) est hydrophile de sorte qu'un effet d'aspiration soit obtenu au travers de la zone d'humidification hydrophile (18) de l'élément d'humidification (10).

4. Unité de contact de capteur conforme à l'une des revendications 1 à 3,
**caractérisée en ce que**
la couche externe (16) est étanche à l'eau à une valeur > 1300 mm, de préférence également imperméable à la vapeur d'eau avec une résistance au passage de la vapeur d'eau Rₑₜ < 20 m²Pa/W.

5. Unité de contact de capteur conforme à l'une des revendications 1 à 4,
**caractérisée en ce que**
la couche interne (14) a une épaisseur de 1 à 50 µm, de préférence de 5 à 15 µm.

6. Unité de contact de capteur conforme à l'une des revendications 1 à 5,
**caractérisée en ce que**
l'élément d'humidification (10) est réalisé imperméable à la vapeur d'eau sur ses faces, de préférence par soudure de la couche interne (14) avec la couche externe (16) dans leur zone externe (22).

7. Unité de contact de capteur conforme à l'une des revendications 1 à 6,
**caractérisée en ce que**
la zone d'humidification (18) de l'élément d'humidification (10) comporte des structures planes en forme de canal qui peuvent être parcourues par un liquide provenant du réservoir de liquide (26).

8. Unité de contact de capteur conforme à la revendication 7,
**caractérisée par**
une membrane d'aération (20) plane ou une soupape d'aération plane positionnée dans la couche interne ou dans la couche externe (14, 16) qui est respectivement reliés aux canaux.

9. Unité de contact de capteur conforme à l'une des revendications 7 et 8,
**caractérisée en ce que**
la configuration plane de la structure en forme de canal incrustée est réalisée sous forme de structures en spirale ou en forme de méandres.

10. Unité de contact de capteur conforme à l'une des revendications 7 à 9,
**caractérisée en ce que**
les épaisseurs de la structure en forme de canal varient, de préférence, les canaux d'alimentation les plus proches du réservoir de liquide sont un peu plus épais que les canaux d'évacuation les plus proches de la membrane d'aération (20) ou de la soupape d'aération.

11. Unité de contact de capteur conforme à l'une des revendications 1 à 10,
**caractérisée en ce que**
le réservoir de liquide (26) comporte une cuve flexible de préférence par exemple en polyéthylène ou en PVC.

12. Unité de contact de capteur conforme à l'une des revendications 1 à 11,
**caractérisée par**
un matériau support textile (32) qui se raccorde de façon plane sur la couche externe.

13. Capteur de thorax comprenant une unité de contact de capteur conforme à l'une des revendications 1 à 12 et une sangle pectorale, le réservoir de liquide (26) étant formé par une cuve flexible et intégré dans la sangle de sorte que par une légère compression de la sangle sur la cuve il soit possible d'exercer une pression qui permet d'obtenir ou d'assister l'humidification de la zone d'humidification (8).
